# EUROPEAN PATENT APPLICATION

(11) **EP 2 194 579 A2**
(43) Date of publication of application: **09.06.2010**
(21) Application number: 08794011.0
(22) Date of filing: 02.07.2008
(51) Int. Cl.: H01L 29/00, A61N 5/02

(54) **DEVICE FOR CONTROLLING PHYSIOLOGICAL PROCESSES IN A BIOLOGICAL OBJECT**

(71) Applicant: Zakrytoe Akcionernoe Obshestvo "Cem Technology", Nizhny Novgorod 603022 (RU)
(72) Inventor: TKACHENKO, Juriy Aleksandrovich, Nizhny Novgorod, 603098 (RU); KOZHEMYAKIN, Aleksandr Mikhailovich, Tomsk, 634050 (RU)
(74) Representative: Dendorfer, Claus
(86) International application number: PCT/RU2008/000282
(87) International publication number: WO 2008/140356

(57) **Abstract**

The invention solves a problem of a non- invasive effect on a biological object for suppressing in it and in its biological substances of pathological factors (pathogenic microorganisms and processes) and/or for stimulating of the biological activity of biological objects and biological substances which constitute the biological object.

The device includes a power source connected to a semiconductor device, the active layer of which contains a semiconductor structure based on A₃B₅ compound. The A₃B₅ semiconductor device is made with a volt-ampere characteristic having a section with a negative differential conductance, the value of which exceeds the differential conductance of the biological object. The semiconductor device may be made as the Gunn diode, as the tunnel diode, as the BARRIT diode and as the transistor. The semiconductor structure may be made, at least, with two working volumes of the same or diverse A₃B₅ compounds with different geometrical dimensions.

## Description

### FIELD OF INVENTION

The invention pertains to devices for exerting electromagnetic effect on a biological object and may be utilized in the medicine and in the veterinary medicine for therapy of a human and animals as well as for changing the biological activity of biological objects.

The invention solves a problem of non- invasive effect on a biological object for suppressing in it and in its biological substances of pathological factors (pathogenic microorganisms and processes) and/or for stimulating of the biological activity of biological objects and biological substances which constitute the biological object. The biological objects include a human, an animal, a living culture while the biological substances include deoxyribonucleic acid (DNA) molecules, organelles, cells, tissues and biological liquids, constituting the biological objects.

### PRIOR ART

Under influence of external factors (including a viral and bacteriological infection, adverse environmental effects, stresses, etc.) a mechanism of biochemical reactions running in the biological substances of biological objects may change which may result in formation in a biological object of conditions favoring evolution of a pathogenic factor provoking disturbance of physiological functions of a biological object.

Application of electromagnetic radiation with wave lengths of the optical (visible and infrared (IR)) band and also of the millimeter (superhigh and extremely high frequencies) and of the centimeter bands is known as a physiotherapeutic treatment of a considerable number of human and animal diseases. The electromagnetic radiation of these bands suppresses evolution of pathological changes and normalizes biochemical activity of biological substances and biochemical reactions running in them. The effect of influence of the electromagnetic radiation on a biological object is being observed at very low power densities not exceeding 10⁻¹⁹ W/cm² (for example, V.I. Petrosyan, E.A. Jeteneva, Yu.A. Gulyayev, "Physics of interaction of millimeter band waves with objects of various nature". Biomedical radio electronics. Radio engineering, 1996, Issue #9, pages 20-31).

The mechanism of influence of the electromagnetic radiation on a state of biological substances of an organism is thought to be linked with the fact that the majority of cells in the structure of biological substances are oscillatory systems, the specter of natural frequencies of which covers ranges spanning from the visible to the centimeter (10⁹-10¹⁵ Hz) wave length bands, while the cells of an organ of a biological substance correspond to a specific range in the organism's irradiation specter (DNA's oscillation frequency is in the range of 2×10⁹ - 9×10⁹ Hz, oscillation frequency of chromosomes and organelle is in the range of 7,5 ×10¹¹ - 1,5× 10¹³ Hz). Natural frequencies of the metabolic processes, including the pathogenic ones, are in the same range. In case the specter of natural frequencies of radiation of biological substances of a biological object coincides with the specter of an external low intensity electromagnetic radiation effecting the said biological object, it gives rise to stimulation of biochemical processes inherent in the biological substances in their normal state, to increasing resistance of the biological object towards external adverse factors, to suppression of pathogenic factors and to adaptation towards environmental changes.

The interaction of an external radiation source with a biological object is of the resonance nature not only in terms of the frequency but also in the radiation power. By increasing power of the external radiation from zero to 10⁻¹⁶-10⁻¹⁹ W/cm², the resonance response of a biological object is being observed (Yu.A. Skripnik, A.F. Yanenko, V.F. Manoilov, etc., Microwave radiometry of physical and biological objects. Zhitomir, Volyn Publishers , 2003, page 63-69), moreover the maximum of the resonance response of relatively weak processes to which, in particular, belong pathologic processes, is observed under considerably lower power values, as compared to processes running in a healthy tissue. Exactly this characteristic property enables to suppress a pathological process with insignificant excitation of a healthy tissue as a background. The effect of the low intensity electromagnetic radiation on a biological object allows to utilize it for physiotherapeutic treatment of a significant number of human and animal deceases, for enhancing stability of a biological object towards adverse external factors and for adaptation to changes in environmental conditions.

There are known devices used for exerting electromagnetic effect on a biological object with the purpose of suppressing a pathogenic factor, such devices contain a voltage source unit, a generator of electromagnetic radiation and an emitting element. The parameters of the electromagnetic radiation (frequency range, power, intensity) are set basing on results of statistic processing of a clinical examination without regarding the individual characteristics of the organism submitted to the therapeutic effect.

The known devices differ from each other in application of generators shaping the emission of a given spectral composition and in aids of matching the generator with the irradiated zone of a biological object.

This way, for example, for shaping a noise-like signal is known a device containing a source of power, outlets of which are connected immediately and via an impulse modulator with the extremely high frequency wave generator based on the avalanche transit-time diode, connected through a ferrite valve and band-pass filter to an antenna emitter (SU2040928, 1995.08.09). For matching the generator with the zone of the biological object subject to the irradiation, as an aid of tuning is used a diaphragm placed in the waveguide (SU1588416, 1990.08.30), a piston (for example, SU1703103, 1992.01.07), etc. The matching element may have a shape of a dielectric plate located between an emitter's aperture and the irradiated zone of a biological object (RU96101587 A, 1998.01.27).

The known devices do not ensure an efficient curative effect by the generated electromagnetic radiation due to a weak correlation of the generated electromagnetic specter with the specter of a pathogenic factor. Besides, these devises are cumbersome and intended for use in steady-state conditions.

There is known a compact portable device consisting of a high frequency signal generator connected to a voltage source, the inlet of the generator is connected to the low frequency generator. The high frequency generator contains a semiconductor active element placed in the strip transmission line resonator; the semiconductor structure of the element is based on the A₃B₅ compound (US6122550, 2000.09.19). The strip line functions as an emitter and during operation is placed on a biological object. In a particular case as an active element is used the Gunn diode.

The shortcoming of this and other known devices is the low efficiency of the curative effect as they generate an electromagnetic radiation with parameters set by the resonance system of the generator according to the statistic data obtained by processing the results of clinical research however disregarding individual characteristics of an organism and spectral characteristic of a pathogenic factor. Besides, while placing the emitter on the surface of the irradiated zone, the biological object "switches in" in the resonance circuit of the generator, thus exerting a not- controlled effect on the parameters of the generated electromagnetic radiation, which causes variations of the specter of the effecting radiation and may incur worsening and evolution of adverse exogenous processes in healthy tissues of an organism.

### DISCLOSURE OF THE INVENTION

The invention is intended for designing a compact radiation generating device, the frequency range of which utmost closely matches the frequency range of a pathogenic factor running in biological substances and in a biological object as a whole at the particular time point.

Furthermore, the invention is also aimed to shape an emission of radiation with parameters individually fitting to each biological object and to a particular effected zone thus matching the state of biological substances (composition on a cellular level, DNA, biophysical and biochemical processes) of a biological object at the time point of formation of this emission.

By solving the abovementioned tasks it becomes possible to exert a more efficient suppression of pathogenic factors in a biological subject as compared to application of so far known devices, to implement an efficient suppression of various a priori unknown pathogenic factors and to promote stimulation of biochemical processes intrinsic to a biological substance in its normal state.

Solution of the abovementioned tasks enhances resistance of a biological object to the adverse external factors, favors suppression of pathogenic factors and adaptation of a biological object to environmental changes.

Similar to already known equipment, the proposed apparatus includes a power source connected to a semiconductor device, the active layer of which contains a semiconductor structure based on A₃B₅ compound; the semiconductor structure is featured by a volt-ampere characteristic having a section with a negative differential conductance, the modulus of which exceeds the value of the differential conductance of a biological object at its natural frequencies (including the natural frequencies of a pathological process) and connected to the open oscillatory circuit.

The semiconductor device may be made as the Gunn diode.

The semiconductor device may be made as the tunnel diode.

The semiconductor device may be made as the BARRIT diode.

The semiconductor device may be made as the transistor.

For enlarging the frequency working range the semiconductor structure may be made at least with two working volumes.

In a particular case the working volumes of the semiconductor structure may be made with diverse geometric dimensions.

In this case the volumes of the semiconductor structure may be made of the same or of diverse A₃B₅ compounds.

In a next particular case the working volumes of the semiconductor structure may be made with the same geometrical dimensions but of diverse A₃B₅ compounds.

It is expedient to utilize the semiconductor structure as the active layer of the Gunn diode and/or of the BARRIT diode and/or of the transistor.

It is expedient to introduce into the device a bi-conductor metal spiral having rounded sections directed towards each other, besides the spiral is placed on a dielectric backing and the semiconductor device is placed in the center of the spiral.

It is expedient to introduce into the device a radiation intensity modulator connected to the control unit placing it between the semiconductor device and a biological object.

The invention is based on characteristic properties of semiconductor structures on the basis of A₃B₅ compounds to which, for example, belong gallium arsenide (GaAs) and indium phosphide (InP), on the basis of which were developed currently widely used such semiconductors as the Gunn diode, the tunnel or BARRIT diode, the transistor, etc. Thanks to these characteristic properties, first of all, it becomes feasible to implement in these structures a volt-ampere characteristic with a negative differential conductance ∂I/∂U < 0 (for example, M.E.Levinstein, Yu.K. Pozhala, M.S. Shur, Gunn effect, Moscow, 1975), the value of which exceeds the differential conductance p of a biological object (|∂I/∂U| > ρ), and, secondly, these structures are inherent in presence of natural electromagnetic oscillations of a background level, the characteristics of which are defined by dipole-active states of volumes of a crystal having defects of the crystal structure at its boundaries.

The value p is different for different biological objects, however for the majority of biological objects it is considerably higher than the differential conductance of the water and is close to the conductance of 0,9% physiological solution NaCI, which makes up not less than 1,5 Cm/m (E.I.Nefedov, A.A.Protopopov, A.A.Khadartsev, A.A.Yashin, Physical and biological principals of information processes in a living substance, Tula, Tula State University, New Medical Technologies Research Institute, 1998); under such conditions formation of an oscillatory process is not possible. However, at the natural frequencies of a biological object the ρ value sharply decreases. During formation of a pathological process in a biological object at its natural frequencies ρ decreases and with ρ<0 in the radiation specter of the biological object there appear the natural frequencies of the pathological process.

Provided the said condition is satisfied, while placing on a biological object (or placing nearby) the A₃B₅ compound basis semiconductor device under a voltage exceeding the threshold U₀ value, which corresponds to the transition to the section with a negative differential conductance on the device's volt- ampere characteristic, the formation of generator circuit with connected biological object (with a semiconductor device placed hereon or nearby) takes place. By doing so, the specter of the generated radiation is determined by the reactive component of the impedance of the "semiconductor device - biological object" system and includes the natural frequencies of the biological substances of the object and of the pathological factor in the affected zone as well as of the biological object is whole, moreover, the generation occurs only at the frequencies of the biological object provided that the condition ∂I/∂U < 0 is satisfied.

The process of generation of the electromagnetic radiation is caused by formation of elevated energy electrons on the cathode of an A₃B₅ compound semiconductor device. The energy of the "hot" electron exceeds by hundreds times the energy of an electron of the conductance zone to which the external voltage is not being supplied. Electrons are drifting in the semiconductor's space and being absorbed by the device's anode. While the elevated energy electrons are crossing the semiconductor and also under conditions of generation of an alternating electromagnetic field linked with the generation process, due to changes of the quantum- mechanical states at its boundary-nearby areas, the dipole-active states of the semiconductor structure are changing. These changes are being determined by an electromagnetic field in a specimen which is formed not only by the semiconductor's volume, but also by the surrounding space including that of the biological object included in the generating circuit of the "semiconductor structure-biological object" system. This way, formation of a stable state of the semiconductor structure with clamping the electromagnetic radiation of the biological object comes about.

After disconnecting the semiconductor device from the power source the altered state of the dipole - active component of the A₃B₅ structure conserves and the characteristics of natural radiation of the structure do not change, they are stable in time provided there are no any external influence with an energy sufficient for causing their changes.

After disconnecting the device from the voltage source, the external electromagnetic field interacts with the dipole- active state of the crystal's volumes resulting in formation of polaritones (bound state of the oscillatory movement of charged particles (dipoles) with an electromagnetic field) (E.A. Vinogradov, "Polaritones of semiconductor microcavity", Successes of physical sciences, Volume 172, Issue #12, 2002, page 1372-1410). The oscillatory state of a crystal of a semiconductor structure and, consequently, of the polaritone's specter is determined by the inside dipole-active state of the structure. While affecting the semiconductor structure by an external electromagnetic radiation by means of the polaritone mechanism, formation of the natural electromagnetic oscillations of a background level in the semiconductor structure comes about; the specter of these oscillations was determined by changes of the dipole-active components of the A₃B₅ structure in the generation mode as well as by the specter of the natural frequencies of a biological object including the pathogenic factor. While using the A₃B₅ semiconductor structure as the generator's active element (while placing it on the biological object and feeding power exceeding the threshold value, i.e. functioning in a mode corresponding to the section with a negative differential conductance of the volt- ampere characteristic) under influence of the external electromagnetic field the state of this structure may alter.

In this case the specter of the repeatedly reflected radiation includes natural frequencies of biological substances and of the biological object in whole. Consequently, the changes in the state of a semiconductor structure and characteristics of its natural radiation are determined also by the specter of own frequencies of the biological object (including a pathogenic factor).

The intensity of the irradiation is weak: the power density does not exceed 10⁻²⁰ W/cm² that is why during the contact of the semiconductor structure with a biological object the radiation does not suppress the biological activity of sound cells of a biological substance in a stable equilibrium condition but suppresses pathological factors which are in the evolution stage and subject to a weak external influence.

Under the influence of an external radiation the trend of stable growth of pathogenic micro organisms and pathogenic processes falls down but the physiological processes under stable equilibrium in the organism's substances are further stabilized. The high efficiency of suppression of the pathogenic factor is caused by the circumstance, that the radiation frequency is correlated with the state of the biological substances and the biological object at the time of formation of effecting irradiation.

This way, if a volt-ampere characteristic of an A₃B₅ compound semiconductor device is featured by a section with a negative differential conductance, the value of which exceeds the value of the differential conductance of a biological object at its natural frequencies, and provided the device is not equipped by a resonator (as a part of device), such conditions allow to shape under external electromagnetic factors an electromagnetic radiation , the specter of which contains natural frequencies of biological substances of a biological object as well as natural frequencies of pathogenic microorganisms and pathological processes.

### EMBODIMENTS

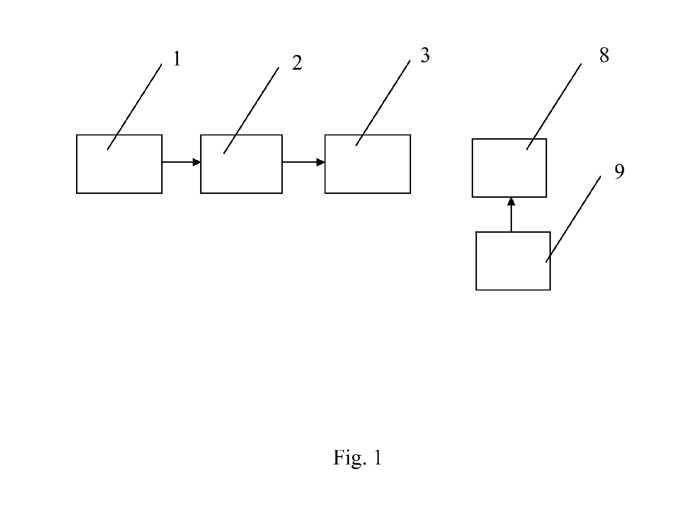

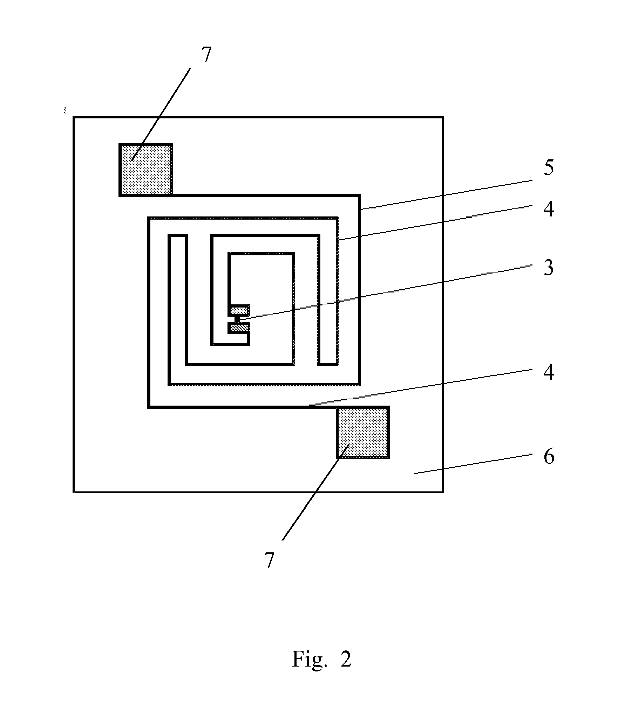

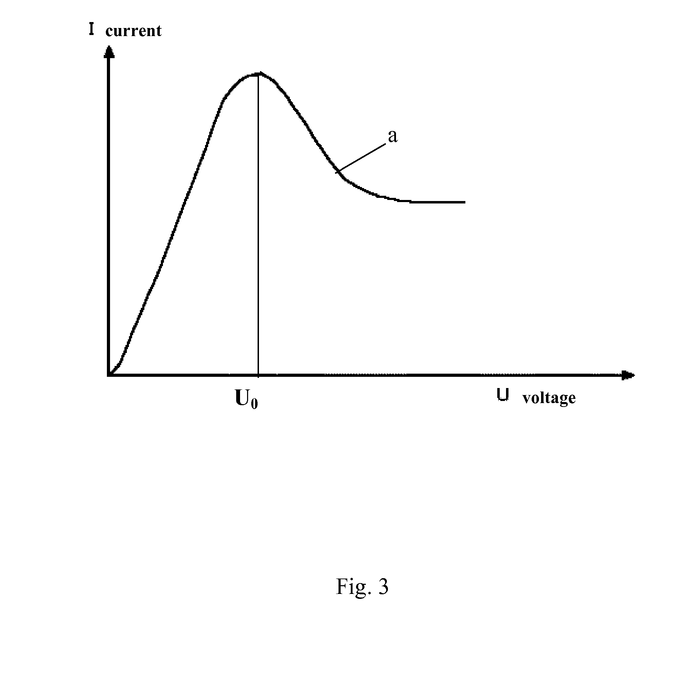

Figure 1 demonstrates the block diagram of the device; figure 2 illustrates one of versions for implementation of the device; figure 3 demonstrates the volt- ampere characteristic of the Gunn diode having an "a" section of a negative differential conductance ∂I/∂U < 0 under voltage values exceeding the threshold value U₀.

The device is equipped with the source of voltage 1, connected through the relay 2 to the semiconductor device 3, which contains a A₃B₅ compound structure. Device 3 performs functions of an active element of the generator, of a receiver of a signal reflected from a biological object in a generation mode as well as a function of a passive transformer of the external radiation with the device disconnected from the power. In the version demonstrated on figure 2, the semiconductor device 3 is located in the center of a bi-conductor metal spiral with spans 4 and 5 rounded and directed towards each other. The spiral and instrument 3 are placed on a dielectric backing 6. The voltage is fed on the spiral through the terminals 7 from the voltage source 1. Application of the bi-conductor spiral enables to use an electromagnetic radiation of a circular polarization (both left- and right- hand polarizations) for exerting an electromagnetic effect on biological substances. It is important for obtaining the best result while affecting the substances, biological components of which are of a spiral configuration and the natural radiation has diverse polarization in different frequency ranges.

The A₃B₅ compound semiconductor structure may be used as the active layer of the Gunn diode and/or of the BARRIT diode and/or of the transistor.

For raising efficiency of effecting pathological factors, in some cases it is expedient to apply in devices 3 various semiconductor structures, which enables to utilize a wider working range of the device.

The semiconductor structure of device 3 may be made with one or several working volumes, while the working volumes may be of different geometrical dimensions and implemented basing on one or several A₃B₅ compounds. Provided the geometrical dimension of the working volumes are equal it is expedient to construct the semiconductor structure of diverse A₃B₅ compounds, but with diverse geometrical dimension of the working volumes the structure may be made of the same A₃B₅ compound. The semiconductor structure may be made from the gallium arsenide (GaAs), from indium phosphide (InP), etc. The common feature which must exist for all A₃B₅ compound-based devices is presence in their volt-ampere characteristic of an "a" section with a negative differential conductance, the value of which exceeds the differential conductance value of a biological object.

If GaAs based device 3 is constructed with one geometrical size of the active layer, it enables theoretically to generate an electromagnetic radiation with frequency band (3 - 70)×10⁹ Hz on the fundamental harmonic. Practically, with specifically defined values of thickness and concentration of conduction electrons of the active layer, this range does not exceed 3-4 octaves. Fabrication of GaAs based device 3 with two working volumes and with diverse dimensions and concentrations of the conduction electrons enables to generate an electromagnetic radiation in a wider frequency band as compared to the first version. Constructing based on GaAs and InP compounds device 3 with diverse geometrical dimensions enables to enlarge the device's working range even more.

To ensure functioning of the device in the mode with the maximum sensitivity of the biological object towards a weak exposure, the device is equipped with the amplitude radiation modulator. In a particular case, the modulator may be constructed as light modulator 8, controlled by control unit 9 and located between instrument 3 and biological object. As modulator 8 may be used a spacer fabricated of the material, transparency of which at working frequencies of the radiator is variable. This spacer may be made as a part of the case of device 3. The spacer may be fabricated of the Schottky barrier semiconductor material, which changes its conductance under an effect of external voltage; may be used also liquid crystal structures controlled by an external electromagnetic radiation, etc. By applying a mechanical modulation of radiation, modulator 8 may be a metal impeller fit on a shaft of a portable electromotor with a low voltage feeding (1,5 - 5 V).The motor's shaft rotational speed may be regulated by means of varying the feeding voltage, which enables additionally to an effect of the main irradiation to exert an influence on a biological object by means of low frequencies.

For therapeutic purposes the device may be applied as for exerting a curative effect on the areas of a human or animal body projected at an affected organ as well as for affecting the reflexotherapy active zones.

Application of the device with a purpose of the therapeutic effect on a biological object proceeds as following.

The device is brought in contact with a biological object and via relay 2 is connected to power source 1, supplying to device 3 the voltage of a value exceeding the threshold value U₀ (fig. 2). By doing so is being formed a generator with a resonance circuit, the impedance of which is linked with the impedance of the biological object in the location of device 3.

Device 3 simultaneously with emitting of a radiation affecting a biological object is receiving an electromagnetic radiation reflected from the biological object, the specter of this radiation contains frequencies corresponding to natural frequencies of a biological substance of the biological object at the time point of its exposure. This radiation changes a state of the device 3 semiconductor structure and, consequently, changes the characteristics of structure's self- radiation in compliance with specific properties of a biological substance. The time interval, during which occurs the variation of the semiconductor structure, is fairly short and doesn't exceed 1 minute.

Thereupon device 3 is being disconnected from the source 1 and left on the biological object at the of area of the previous exposure (device's passive mode), while the biological object is being affected by a radiation, the specter of which corresponds to the radiation frequency range of the biological object subject to an active working mode of device 3 and to natural frequencies of pathogenic factors. This action results in generating of conditions adverse for evolution of pathogenic microorganisms and pathologic processes, while the conditions for the vital activity of sound cells and organelles of a biological object and its physiological functions do not undergo adverse changes.

The device enables to suppress pathogenic microorganisms and pathological processes emerging in a biological object under influence of various external factors in their early stages of evolution and ensures an efficient treatment of variety of diseases.

### INDUSTRIAL APPLICABILITY

The device is manufactured on the basis of elements currently produced by the industry.

The device was tested for treatment of diseases of the respiratory apparatus, of the gastrointestinal tract, of the musculoskeletal system, of the nervous system, of children's atopic dermatitis, of inflectional diseases (including viral one), etc. The biological substance, exposed to the electromagnetic radiation, is the human body surface, affected itself or projected at an organ affected by a pathological factor (for example, at the basis of spinal roots during treatment of osteochondrosis, at tonsils' area during treatment or prevention of influenza and acute respiratory disease, at trachea's area during treatment of breathing organs). The time of effecting the human by an external radiation in the active mode of the device (by connecting structure 2 to voltage source 1) does not exceed 1-2 minutes; in the passive mode of the device (by disconnecting structure 2 from voltage source 1) the time of exposure by the electromagnetic radiation emitted by device 3 under influence of natural electromagnetic background or by man-made source of radiation varied from 10 minutes to 1,5 hour, it was noted besides that the treatment response took place in all cases. Below are reported results of the performed research.

47 patients (18 men and 29 women) aged between 32 and 69 years were observed during clinical treatment of a pain syndrome of vertebrogenic character using the developed device. All the patients were suffering from osteochondrosis of various parts of the vertebrae. For 14 patients the vertebrae osteochondrosis with radicular syndrome was the fundamental illness, for the rest of patients it was an associated illness. As a result of the treatment the acute pain syndrome was completely relieved in 1-3 days, the rest of the patients noted considerable pain relief and change of its localization , the patients felt much better (improvements of the locomotive activity and of the limbs blood circulation, disappearance of headache). After 5-10 days by 45 patients (95%) the pain syndrome was observed to disappear completely; it worth to mention that in 45% of cases the pain disappeared for a term of exceeding 1 year.

The device for tested for treatment of root and reflector vertebrae osteochondrosis manifestations including concomitant somatic pathology for 48 patients aged between 37 and 72 years. The evaluation of the treatment effectiveness was implemented basing on patients' subjective sensation and on the electroneuromyographic examination. The relief of painful sensation and qualitative (the pain transformed to dull and boring sensation losing lumbago gradation) was observed for all patients after 30 minutes, a relief of vertebrae and limb joint stiffness as well as improvement of muscle weakness was also reported. After 6 conducted treatments for 43 patients the pain syndrome disappeared, while measuring the dermal temperature at the innervated areas of affected roots the thermal asymmetry was observed to diminish considerably (before treatment it was 3,0-4,0°C, after the treatment approximately 0,6°C), an essentially positive trend of amplitude and velocity parameters of the neuromuscular apparatus was noted, the regional hemodynamics was noted to improve. For patients with concomitant osteoarthritis it was noted a noticeable diminishing of the pain syndrome and of an edema under an affected joint, the stiffness improved, the range of active painless movements increased.

Application of the device for treatment of children's localized erythematic atopic dermatitis with lichenification has enabled to achieve in shorter terms as compared to a pharmacotherapy a clinical remission of the disease, to expedite infiltration resorption dispersal and skin regeneration processes. 49 patients suffering from atopic dermatitis of localized and extensive form and of light and middle severity were under examination. All patients were administered enterogel, 37 patients were additionally exposed by an electromagnetic field in accordance with the developed method. Prior and after the treatment patients of all groups were clinically studied with evaluation of the symptoms using the SCORAD scale. The paraclinic examination included common blood and urine analyses, biochemical blood analysis with determination of the blood protein, transaminases (alaninaminotransferase, aspartataminatransferase), bilirubin, glucose, thymole test, immune status (T-helpers,T-supressors, T-lymphocites, B-lymphocites, circulation immune complexes, immunoglobulines - IgA, IgM, IgG, IgE). For objectification of the affected dermal areas the lesion focuses were examined by an eritemo-melaninometer EMM-O1, the instrument being designated for determination of erythema manifestation degree and human skin pigmentation degree by means of selective spectral measurements of reflection coefficients and calculation of erythema and skin pigmentation indexes. For all patients at completion of the treatment was observed a positive trend of clinical symptoms, besides the best trend for clinical symptoms was noted in a group treated by the developed method. The treatment direct efficiency by the declared method accounted for 92,5%, the preservation of the therapeutic effect is not less that 11 months while for the patients treated by pharmacotherapy the preservation lasts 3-4 months.

The device was tested during an influenza epidemic for reducing the risk of illness. 24 persons aged between 21 and 65 years took part in the conducted studies. For 2 persons of this group light form symptoms of an acute respiratory viral infection were observed. 1 person became ill with acute sickness manifestations (intense throat pain, coughing, high temperature), however these symptoms disappeared after 20 hours. The rest members of the group remained healthy.

The device is compact and may be used as in stationary as well as in ambulance and home conditions. The device is classified as a human safe instrument, as the working voltage of all elements does not exceed 15 V.

## Claims

1. The device for regulating physiological processes in a biological object containing a power source connected to a semiconductor device, the active layer of which contains a semiconductor structure based on A₃B₅ compound; the semiconductor A₃B₅ device is distinguishing by a volt-ampere characteristic having a section with a negative differential conductance, the modulus of which exceeds the value of the differential conductance of the biological.

2. According to claim 1, the device is distinguished by the semiconductor device made as the Gunn diode.

3. According to claim 1, the device is distinguished by the semiconductor device made as the tunnel diode.

4. According to claim 1, the device is distinguished by the semiconductor device made as the BARRIT diode.

5. According to claim 1, the device is distinguished by the semiconductor device made as the transistor.

6. According to claim 1, the device is distinguished by the semiconductor structure made at least with two working volumes.

7. According to claim 6, the device is distinguished by the working volumes of the semiconductor structure may be made with diverse geometric dimensions.

8. According to claim 6, the device is distinguished by the working volumes of the semiconductor structure may be made of the same or diverse A₃B₅ compounds.

9. According to claim 6, the device is distinguished by the working volumes of the semiconductor structure may be made with the same geometric dimensions of diverse A₃B₅ compounds.

10. According to any of the claims 6-9, the device is distinguished by the semiconductor structure as is applied as the active layer of the Gunn diode and/or of the BARRIT diode and/or of the transistor.

11. According to any of the claims 1-10, the device is distinguished by containing a bi-conductor metal spiral with rounded sections directed towards each other, besides the spiral is placed on a dielectric backing while the semiconductor device is placed in the center of the spiral.

12. According to any of the claims 1-11, the device is distinguished by a built-in an irradiation intensity modulator connected to the control unit and placed in front of the semiconductor device.

13. According to any of the clams 1-12, the device is distinguished itself that it is applied for therapy of a human and animals as well as for changing the biological activity of various biological objects.
